(19) Europäisches Patentamt
European Patent Office
Office européen des brevets

(11) Publication number: 0 236 902 B1

(12) EUROPEAN PATENT SPECIFICATION

(45) Date of publication of patent specification: 05.12.90

(51) Int. Cl.[5]: C 07 D 239/22, C 07 D 401/06, C 07 D 413/04, A 61 K 31/505

(21) Application number: 87102922.9

(22) Date of filing: 02.03.87

# (54) 1,2,3,4-tetrahydro-6-substituted-4-aryl(or heterocyclo)-3-substituted-2-thioxo (or oxo)-5-pyrimidinecarboxylic acids and esters.

(30) Priority: 14.03.86 US 839770
14.03.86 US 840130

(43) Date of publication of application:
16.09.87 Bulletin 87/38

(45) Publication of the grant of the patent:
05.12.90 Bulletin 90/49

(84) Designated Contracting States:
BE CH DE FR GB IT LI NL SE

(56) References cited:
GB-A-1 561 290
US-A-4 041 035
US-A-4 609 494

(73) Proprietor: E.R. Squibb & Sons, Inc.
Lawrenceville-Princeton Road
Princeton, N.J. 08540-4000 (US)

(72) Inventor: Atwal, Karnail
2634 Old Stone Mill Drive
Cranbury, NJ (US)

(74) Representative: Staub, Gabriella, Dr. et al
MODIANO, JOSIF, PISANTY & STAUB Via
Meravigli, 16
I-20123 Milan (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 236 902 B1

**Description**

Compounds having the formula I

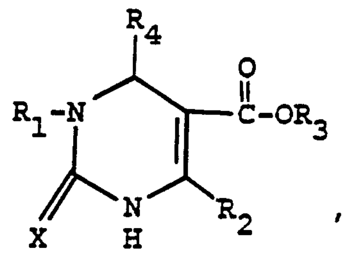

and pharmaceutically acceptable salts thereof, are cardiovascular agents. In formula I, and throughout the specification, the symbols are as defined below.

X is oxygen or sulfur;

$R_1$ is alkyl, cycloalkyl, alkenyl, alkynyl, aryl, $-(CH_2)_n-Y_2$, $-(CH_2)_p-Y_3$, halo substituted alkyl, or

$$-\overset{O}{\underset{O}{\overset{\|}{\underset{\|}{S}}}}-Y_4;$$

$R_2$ is hydrogen, alkyl, alkenyl, alkynyl, cycloalkyl, aryl, $-(CH_2)_n-Y_1$, or halo substituted alkyl;

$R_3$ is hydrogen, alkyl, cycloalkyl, aryl, heterocyclo, $-(CH_2)_n-Y_2$, $-(CH_2)_p-Y_3$, or halo substituted alkyl;

$R_4$ is aryl or heterocyclo;

$Y_1$ is cycloalkyl, aryl, heterocyclo, hydroxyl, alkoxy, aryl-$(CH_2)_m-O-$, mercapto, alkylthio, aryl$-(CH_2)_m-S-$, amino, substituted amino, carbamoyl,

$$\text{(substituted amino)-}\overset{O}{\overset{\|}{C}}-,\quad \text{heterocyclo-}(CH_2)_m-\overset{O}{\overset{\|}{C}}-,$$

carboxyl, alkoxycarbonyl,

$$\text{alkyl-}\overset{O}{\overset{\|}{C}}-,\quad \text{aryl-}(CH_2)_m-\overset{O}{\overset{\|}{C}}-,\quad \text{alkyl-}\overset{O}{\overset{\|}{C}}-O-\text{ or }\ \text{aryl-}(CH_2)_m-\overset{O}{\overset{\|}{C}}-O-;$$

$Y_2$ is cycloalkyl, aryl, heterocyclo, carbamoyl,

$$\text{(substituted amino)-}\overset{O}{\overset{\|}{C}}-,$$

carboxyl, alkoxycarbonyl,

$$\text{alkyl-}\overset{O}{\overset{\|}{C}}-,\quad \text{aryl-}(CH_2)_m-\overset{O}{\overset{\|}{C}}-\text{ or }\ \text{heterocyclo}(CH_2)_m-\overset{O}{\overset{\|}{C}}-;$$

$Y_3$ is hydroxyl, alkoxy, aryl-$(CH_2)_m-O-$, mercapto, alkylthio, aryl-$(CH_2)_m-S-$,

$$\text{alkyl-}\overset{O}{\overset{\|}{C}}-O-,\quad \text{aryl-}(CH_2)_m-\overset{O}{\overset{\|}{C}}-O-,$$

amino, or substituted amino;

$Y_4$ is alkyl, cycloalkyl, aryl, heterocyclo, $-(CH_2)_n-Y_1$ or halo substituted alkyl;

m is 0 or an integer of 1 to 6;

n is an integer of 1 to 6; and

p is an integer of 2 to 6.

Listed below are definitions of various terms used to describe the compounds of this invention. These

definitions apply to the terms as they are used throughout the specification (unless they are otherwise limited in specific instances) either individually or as part of a larger group.

The terms "alkyl" and "alkoxy" refer to both straight and branched chain groups. Those groups having 1 to 8 carbon atoms are preferred.

The term "halo substituted alkyl" refers to alkyl groups (as described above) in which one or more hydrogens have been replaced by chloro, bromo or fluoro groups. Exemplary groups are trifluoromethyl, which is preferred, pentafluoroethyl, 2,2,2-trichloroethyl, chloromethyl, bromomethyl, etc.

The term "aryl" refers to phenyl and substituted phenyl. Exemplary substituted phenyl groups are phenyl groups substituted with one, two or three alkyl, alkoxy, alkylthio, halo, nitro, cyano, hydroxy, amino, alkylamino, dialkylamino, trifluoromethyl, isothiocyanato, isocyanato, or difluoromethoxy groups.

The terms "alkenyl" and "alkynyl" refer to both straight and branched chain groups. Those groups having 2 to 8 carbon atoms are preferred.

The term "cycloalkyl" refers to those groups having 3, 4, 5, 6 or 7 carbon atoms.

The term "halo" refers to chloro, bromo, fluoro and iodo.

The term "heterocyclo" refers to fully saturated or unsaturated rings of 5 or 6 atoms containing one or two oxygen or sulfur atoms and/or one to four nitrogen atoms provided that the total number of hetero atoms in the ring is 4 or less. The heterocyclo ring is attached by way of an available carbon atom. Preferred monocyclic heterocyclo groups include 2- and 3-thienyl, 2- and 3-furyl, 2- and 3-pyrrolyl, 2-, 3- and 4-pyridyl, 2-, 4- and 5-imidazolyl, 2- and 3-pyrrolidinyl, 2-, 3- and 4-piperidinyl, and 2-, 3- and 4-azepinyl. The term heterocyclo also includes bicyclic rings wherein the five or six membered ring containing oxygen, sulfur and nitrogen atoms as defined above is fused to a benzene ring and the bicyclic ring is attached by way of an available carbon atom in the benzene ring. Preferred bicyclic heterocyclo groups include 4, 5, 6 or 7-indolyl, 4, 5, 6 or 7-isoindolyl, 5, 6, 7 or 8-quinolinyl, 5, 6, 7 or 8-isoquinolinyl, 4, 5, 6 or 7-benzothiazolyl, 4, 5, 6 or 7-benzoxazolyl, 4, 5, 6 or 7-benzimidazolyl, 4, 5, 6 or 7-benzoxadiazolyl, and 4, 5, 6 or 7-benzofurazanyl.

The term heterocyclo also includes such monocyclic and bicyclic rings as defined above substituted with one, or more, alkyl, arylalkyl, diarylalkyl, alkylthio, alkoxy, halo, nitro, oxo, cyano, hydroxy, amino, alkylamino, dialkylamino, trifluoromethyl, isocyanato, isothiocyanato or difluoromethoxy groups.

The term "substituted amino" refers to a group of the formula $-NZ_1Z_2$ wherein $Z_1$ is hydrogen, alkyl, or aryl-$(CH_2)_m-$ and $Z_2$ is alkyl or aryl-$(CH_2)_m-$ or $Z_1$ and $Z_2$ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, or 1-pyrrolidinyl, 1-piperidinyl, or 1-azepinyl substituted with alkyl, alkoxy, alkylthio, halo, trifluoromethyl or hydroxy.

Detailed Description of the Invention

The compounds of formula I, and the pharmaceutically acceptable salts thereof, are cardiovascular agents. They act as calcium entry blocking vasodilators and are especially useful as hypotensive agents. Thus, by the aministration of a composition containing one (or a combination) of the compounds of this invention, the blood pressure of a hypertensive mammalian (e.g., human) host is reduced. A single dose, or two to four divided daily doses, provided on a basis of about 0.1 to 100 milligrams per kilogram of body weight per day, preferably from about 1 to about 50 milligrams per kilogram per day, is appropriate to reduce blood pressure. The substance is preferably administered orally, but parenteral routes such as the subcutaneous, intramuscular or intravenous routes can also be employed.

As a result of the calcium entry blocking activity of the compounds of formula I, and the pharmaceutically acceptable salts thereof, it is believed that such compounds in addition to being hypotensive agents may also be useful as antiarryhythmic agents, anti-anginal agents, anti-fibrillatory agents, anti-asthmatic agents, and in limiting myocardial infarction.

The compounds of this invention can also be formulated in combination with a diuretic, or a beta-adrenergic agent, or angiotensin converting enzyme inhibitor. Suitable diuretics include the thiazide diuretics such as hydrochlorothiazide and bendroflumethiazide, suitable beta-adrenergic agents include nadolol, and suitable angiotensin converting enzyme inhibitors include captopril.

The compounds of formula I can be formulated for use in the reduction of blood pressure in compositions such as tablets, capsules or elixirs for oral administration, or in sterile solutions or suspensions for parenteral administration. About 10 to 500 milligrams of a compound of formula I is compounded with physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

The compounds of formula I wherein X is sulfur can be prepared by reacting a keto ester compound having the formula

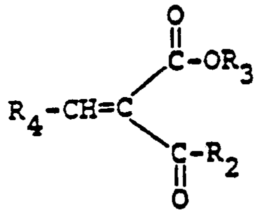

II

with an S-(phenylmethyl)thiopseudourea having the formula

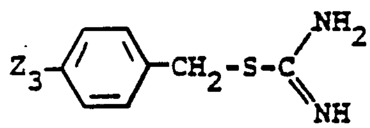

III

or a salt thereof. In formula III, and throughout the specification, $Z_3$ is hydrogen or methoxy. The reaction mixture is heated in the presence of sodium acetate to yield a tautomeric mixture of compounds having the formulas

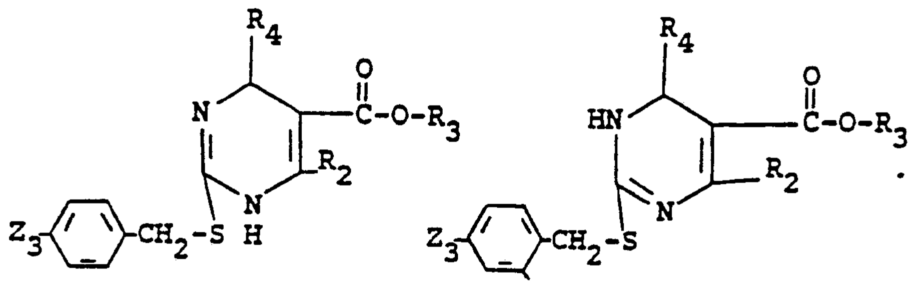

IV

Reaction of a tautomeric mixture of formula IV with a compound having the formula V

$R_1$-halogen

in the presence of an inorganic base yields the corresponding compound having the formula VI

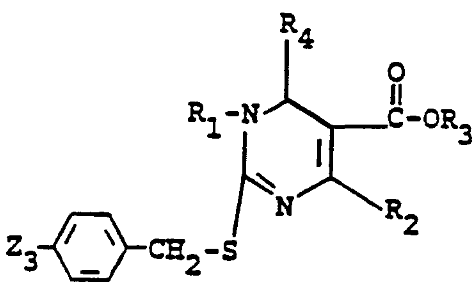

VI

A compound of formula VI wherein $Z_3$ is hydrogen can be converted to the corresponding product of formula I wherein X is sulfur by treatment with bromotrimethylsilane. A compound of formula VI wherein $Z_3$ is methoxy can be converted to the corresponding product of formula I wherein X is sulfur by treatment with trifluoroacetic acid and ethanethiol.

The compounds of formula I wherein X is oxygen can be prepared by heating a keto ester of formula II with O-methylpseudourea

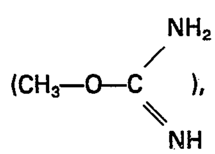

or a salt thereof, in the presence of sodium acetate or sodium bicarbonate to yield a tautomeric mixture of compounds having the formulas

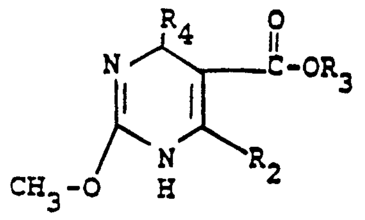 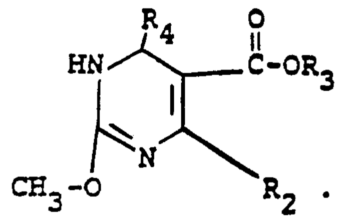 . VII

Reaction of a tautomeric mixture of formula VII with a compound of formula V in the presence of an inorganic base yields the corresponding compound having the formula

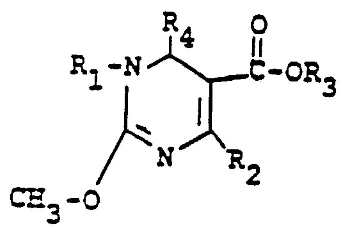 . VIII

A compound of formula VIII can be converted to the corresponding product of formula I wherein X is oxygen by treatment with hydrochloric acid.

In those instances wherein the reactants described above contain reactive substituents not meant to participate in the reaction, it may be necessary to first protect these functional groups, carry out the desired reaction, and then remove the protecting group.

The compounds of formula I that contain a basic or acid group form acid addition and basic salts with a variety of inorganic and organic acids and bases. The pharmaceutically acceptable salts are preferred, although other salts may also be useful in isolating or purifying the product. Such pharmaceutically acceptable acid addition salts include those formed with hydrochloric acid, methanesulfonic acid, toluenesulfonic acid, sulfuric acid, acetic acid, maleic acid, etc. Pharmaceutically acceptable basic salts include alkali metal salts (e.g., sodium, potassium and lithium) and alkaline earth metal salts (e.g., calcium and magnesium). The salts can be obtained by reacting the product with an equivalent amount of the acid in a medium in which the salt precipitates.

Preferred compounds of this invention are those wherein:

$R_2$ is alkyl (especially methyl), $R_3$ is alkyl (especially ethyl) and $R_4$ is 3-nitrophenyl.

The following examples are specific embodiments of this invention.

### Example 1

1,2,3,4-Tetrahydro-6-methyl-4-(3-nitrophenyl)-2-oxo-3-(3-phenylpropyl)-5-pyrimidinecarboxylic acid, 1-methylethyl ester

A) 1,4-Dihydro-2-methoxy-6-methyl-4-(3-nitrophenyl)-5-pyrimidinecarboxylic acid, 1-methylethyl ester

A reaction mixture containing 2-[(3-nitrophenyl)methylene]-3-oxobutanoic acid, 1-methylethyl ester (10.0 g, 36.0 mmol), sodium bicarbonate (8.40 g, 108 mmol), and O-methylpseudourea hydrogen sulfate (8.06 g, 46.8 mmol) in dimethylformamide (54 ml) was heated at 60°C under argon for about 2½ days. The reaction mixture was diluted with water and extracted with ethyl acetate. The organic phase was washed with water (six times) and saturated sodium chloride, dried (potassium carbonate) and evaporated. The residue was pressed through a short pad of silica gel and crystallized from isopropyl ether/hexane to give the title compound as yellow crystals (8.04 g).

B) 1,6-Dihydro-2-methoxy-6-methyl-4-(3-nitrophenyl)-1-(3-phenylpropyl)-5-pyrimidinecarboxylic acid, 1-methylethyl ester

A solution of 1,4-dihydro-2-methoxy-6-methyl-4-(3-nitrophenyl)-5-pyrimidinecarboxylic acid, 1-methylethyl ester (4.0 g, 12.0 mmol) in dry dimethylformamide (10 ml) under argon was treated with finely ground potassium carbonate (4.97 g, 36.0 mmoles), 3-phenylpropyl bromide (2.19 ml, 14.4 mmoles) and a catalytic amount of 18-crown-6. The resulting suspension was allowed to stir at room temperature for 72 hours, diluted with ether, filtered and the filtrate washed with water and brine. After drying over anhydrous magnesium sulfate, the solvent was evaporated. The residue was purified by flash chromatography (10—15% ethyl acetate in hexanes) to provide the desired product (3.29 g) as a yellow oil.

C) 1,2,3,4-Tetrahydro-6-methyl-4-(3-nitrophenyl)-2-oxo-3-(3-phenylpropyl)-5-pyrimidinecarboxylic acid, 1-methylethyl ester

A solution of 1,6-dihydro-2-methoxy-6-methyl-4-(3-nitrophenyl)-1-(3-phenylpropyl)-5-pyrimidinecarboxylic acid, 1-methylethyl ester (1.96 g, 4.34 mmoles) in methanol (20 ml) was treated with 2.5 N hydrochloric acid (5 ml) and the resulting mixture was allowed to stir at room temperature overnight. A

colorless solid precipitated out. Methanol was evaporated and the residue was taken up in ethyl acetate. The solution was washed with water, sodium bicarbonate solution and brine. It was dried over anhydrous magnesium sulfate and evaporated. The residue was crystallized from dichloromethane/isopropyl ether to provide the title compound as a colorless solid (1.61 g), melting point 149.5—151.5°C.

| | | | |
|---|---|---|---|
| Analysis Calc'd. for $C_{24}H_{27}N_3O_5$: | C, 65.89; | H, 6.22; | N, 9.60 |
| Found: | C, 66.05; | H, 6.28; | N, 9.60 |

Example 2

1,2,3,4-Tetrahydro-6-methyl-4-(3-nitrophenyl)-2-oxo-3-(2-propenyl)-5-pyrimidinecarboxylic acid, 1-methylethyl ester

A) 1,6-Dihydro-2-methoxy-6-methyl-4-(3-nitrophenyl)-1-(2-propenyl)-5-pyrimidinecarboxylic acid, 1-methylethyl ester

A solution of 1,4-dihydro-2-methoxy-6-methyl-4-(3-nitrophenyl)-5-pyrimidinecarboxylic acid, 1-methylethyl ester (4.0 g, 12.0 mmol; see Example 1A) in dry dimethylformamide (10 ml) was treated with finely ground potassium carbonate (6.6 g, 48.0 mmoles) and allyl bromide (1.7 ml, 20.0 mmole). The resulting suspension was allowed to stir under argon at room temperature for 10 hours. The reaction was diluted with ethyl acetate, filtered and the filtrate was washed with water and brine. It was dried over anhydrous magnesium sulfate and evaporated to provide a yellow oil. Purification by flash chromatography (20% ethyl acetate in hexanes) yielded the title compound (2.64 g) as a yellow oil.

B) 1,2,3,4-Tetrahydro-6-methyl-4-(3-nitrophenyl)-2-oxo-3-(2-propenyl)-5-pyrimidinecarboxylic acid, 1-methylethyl ester

A solution of 1,6-dihydro-2-methoxy-6-methyl-4-(3-nitrophenyl)-1-(2-propenyl)-5-pyrimidinecarboxylic acid, 1-methylethyl ester (1.44 g, 3.86 mmol) in methanol (10 ml) was treated with 2.5 N hydrochloric acid (30 ml) and the reaction was allowed to stir at room temperature for 10 hours. By the end of this period, a colorless precipitate was formed. The reaction was diluted with ethyl acetate and the organic layer was separated. The aqueous layer was reextracted with ethyl acetate and the combined organic extracts were washed with sodium bicarbonate and brine. After drying over anhydrous magnesium sulfate, the solvent was evaporated to provide a colorless solid. It was triturated with isopropyl ether and was filtered (1.07 g). Recrystallization from dichloromethane-isopropyl ether gave the title compound (975 mg) as a colorless solid, melting point 172—174°C.

| | | | |
|---|---|---|---|
| Analysis Calc'd. for $C_{18}H_{21}N_3O_5$: | C, 60.16; | H, 5.89; | N, 11.69 |
| Found: | C, 60.08; | H, 5.83; | N, 11.65 |

Example 3

1,2,3,4-Tetrahydro-6-methyl-3-[3-[methyl(phenylmethyl)amino]propyl]-4-(3-nitrophenyl)-2-oxo-5-pyrimidinecarboxylic acid, 1-methylethyl ester, monohydrochloride

A) N-Benzyl-3-chloro-N-methylpropylamine

A solution of N-benzyl-N-methylpropanol (25.0 g, 139.5 mmol) in chloroform (50 ml) was cooled in an ice bath and was treated dropwise with methanolic hydrochloric acid (150 ml of a 1N solution). After the addition was finished, the cooling bath was removed, and the solution was allowed to warm to room temperature. The solvent and excess hydrochloric acid were removed under reduced pressure to yield a thick oil. This was dissolved in chloroform (25 ml), cooled to 0°C and was treated dropwise with thionyl chloride (30 ml). After the addition was complete, the reaction was allowed to warm to room temperature and then heated at 70°C for 3 hours. The reaction was allowed to cool to ambient temperature and the solvent was removed *in vacuo*. The residue was partitioned between ether/chloroform (80:20) and 2N sodium hydroxide. The organic layer was separated and the aqueous layer was reextracted with the same solvent system. The combined extracts were washed with brine, dried over anhydrous magnesium sulfate and evaporated to provide a yellow oil (26.1 g).

B) 1,6-Dihydro-2-methoxy-6-methyl-1-[3-[methyl(phenylmethyl)amino]propyl]-4-(3-nitrophenyl)-5-pyrimidinecarboxylic acid, 1-methylethyl ester

A solution of 1,4-dihydro-2-methoxy-6-methyl-4-(3-nitropenyl)-5-pyrimidinecarboxylic acid, 1-methylethyl ester (2.0 g, 6.0 mmol) in dimethylformamide (7.0 ml) was treated with finely ground potassium carbonate (1.7 g, 12.0 mmoles), N-benzyl-3-chloro-N-methylpropylamine (2.37 g, 12.0 mmol) and a catalytic amount of 18-crown-6. The reaction was heated at 70—75°C under argon overnight. The reaction was allowed to cool down to room temperature and was diluted with ether. It was filtered, and the filtrate was washed with water, brine and was dried over anhydrous magnesium sulfate. Evaporation of solvent provided a brown oil which was purified by flash chromatography (20% acetone in hexanes) to yield the title compound (1.15 g) as a yellow oil.

C) 1,2,3,4-Tetrahydro-6-methyl-3-[3-[methyl(phenylmethyl)amino]propyl]-4-(3-nitrophenyl)-2-oxo-5-pyrimidinecarboxylic acid, 1-methylethyl ester, monohydrochloride

A solution of 1,6-dihydro-2-methoxy-6-methyl-1-[3-[methyl(phenylmethyl)amino]propyl]-4-(3-nitro-

phenyl)-5-pyrimidinecarboxylic acid, 1-methylethyl ester (1.50 g, 3.0 mmol) in methanol (12.0 ml) was treated with 2.5 N hydrochloric acid (5.0 ml). The reaction was allowed to stir at room temperature for 16 hours. The solvent was evaporated and the residue was treated with sodium hydroxide and extracted with dichloromethane. The combined extracts were washed with brine and dried over anhydrous magnesium sulfate. The solvent was evaporated and the residue in dichloromethane was converted into the hydrochloric acid salt. The solvent was removed and the residue was crystallized from acetonitrile-ether to provide colorless solid (1.2 g). Recrystallization from the same solvent system provided the analytically pure title compound (1.08 g), melting point 165—170°C.

| | | | | |
|---|---|---|---|---|
| Analysis Calc'd. for $C_{26}H_{32}N_4O_5 \cdot HCl$: | C, 60.40; | H, 6.43; | N, 10.83; | Cl, 6.86 |
| Found: | C, 60.16; | H, 6.39; | N, 10.79; | Cl, 6.78 |

Example 4

1,2,3,4-Tetrahydro-6-methyl-4-(3-nitrophenyl)-3-(2-propenyl)-2-thioxo-5-pyrimidinecarboxylic acid, methyl ester

A) S-(4-Methoxybenzyl)thiopseudourea, hydrochloride

A suspension of thiourea (38 g, 50.0 mmole) in dry tetrahydrofuran (40 ml) was cooled to 0°C under argon and was treated dropwise with 4-methoxybenzylchloride (8.0 g, 50.0 mmole). After the addition was completed, the cooling bath was removed and the reaction was allowed to stir at room temperature for 2 hours. It was then heated at 60—65°C for 4 hours whereupon a colorless voluminous precipitate was formed. The reaction was allowed to cool down to room temperature and was diluted with anhydrous ether. The solid was filtered off and washed with anhydrous ether to give 10.92 g of 2-(4-methoxybenzyl)-2-thiopseudourea, hydrochloride, melting point 161—163.5°C.

| | | | | | |
|---|---|---|---|---|---|
| Analysis Calc'd. for $C_9H_{12}N_2OS \cdot HCl$: | C, 46.45; | H, 5.63; | N, 12.04; | S, 13.78; | Cl, 15.23 |
| Found: | C, 46.48; | H, 5.64; | N, 12.25; | S, 13.74; | Cl, 15.31 |

B) 1,4-Dihydro-2-[[(4-methoxyphenyl)methyl]thio]-6-methyl-4-(3-nitrophenyl)-5-pyrimidinecarboxylic acid, methyl ester

A solution of 2-[(3-nitrophenyl)methylene]-3-oxobutanoic acid, methyl ester (5.0 g, 0.02 mole) in 20 ml of dimethylformamide under argon at room temperature was treated with S-(4-methoxybenzyl)-S-thiopseudourea, hydrochloride (4.65 g, 0.02 mole) and sodium acetate (1.64 g, 0.02 mole). The mixture was then heated at 65 ± 5°C for 3 hours. Upon cooling, ethyl acetate was added and a small amount of solids were filtered. The filtrate was washed with water (twice), aqueous sodium bicarbonate and saturated brine. The aqueous washes were extracted with fresh ethyl acetate. The combined filtrate and washings were dried (magnesium sulfate) and concentrated *in vacuo* to give about 9 g of crude product. Crystallization from acetone-isopropyl ether gave 6.8 g of product, melting point 125—127.5°C, tlc, silica gel, ethyl acetate/hexane (1:1), $R_f = 0.48$.

| | | | | |
|---|---|---|---|---|
| Analysis Calc'd. for $C_{21}H_{21}N_3O_5S$: | C, 59.00; | H, 4.95; | N, 9.83; | S, 7.50 |
| Found: | C, 58.86; | H, 4.82; | N, 9.51; | S, 7.25 |

C) 1,6-Dihydro-2-[[(4-methoxyphenyl)methyl]thio]-4-methyl-6-(3-nitrophenyl)-1-(2-propenyl)-5-pyrimidinecarboxylic acid, methyl ester

A slurry of sodium hydride (168 mg, 4.2 mmole, 60% in mineral oil dispersion) in 5 ml of dry tetrahydrofuran at 0°C under argon was treated dropwise with 1,4 - dihydro - 2 - [[(4 - methoxyphenyl)-methyl]thio] - 6 - methyl - 4 - (3 - nitrophenyl) - 5 - pyrimidinecarboxylic acid, methyl ester in 15 ml of dry tetrahydrofuran. After an additional 10 minutes at 0°C, allyl bromide was added and the reaction mixture was allowed to warm to room temperature overnight.

Tetrahydrofuran was removed *in vacuo* and the residue, dissolved in ethyl acetate, was washed with 1N hydrochloric acid, water (twice), aqueous sodium bicarbonate, water and saturated brine. The aqueous fractions were extracted with fresh ethyl acetate. The combined organic fractions were dried (magnesium sulfate) and concentrated *in vacuo* to give 1.5 g of crude oily product. Flash chromatography on 250 ml of silica gel and elution with ethyl acetate/hexanes (1:4) gave 1.0 g of the title compound as an oil.

D) 1,2,3,4-Tetrahydro-6-methyl-4-(3-nitrophenyl)-3-(2-propenyl)-2-thioxo-5-pyrimidinecarboxylic acid, methyl ester

1,6 - Dihydro - 2 - [[(4 - methoxyphenyl)methyl]thio] - 4 - methyl - 6 - (3 - nitrophenyl) - 1 - (2 - propenyl) - 5 - pyrimidinecarboxylic acid, methyl ester (1.0 g, 2.14 mmol) in 15 ml of dichloromethane under argon at room temperature was treated with trifluoroacetic acid (0.6 ml, 0.85 g, 7.7 mmole) and ethanethiol (0.4 ml, 0.33 g, 5.4 mmole). No change (tlc) occurred within 2 hours. Heating at reflux temperature, however, effected complete reaction in several hours.

Volatiles were evaporated *in vacuo* and the residue (solidified) was triturated with isopropyl ether to give 0.65 g of off-white powder, melting point 171.5—173.0°C. Crystallization from acetone/isopropyl ether afforded 450 mg of the title compound, melting point 175—177°C.

| | | | | |
|---|---|---|---|---|
| Analysis Calc'd. for $C_{16}H_{17}N_3O_4S$: | C, 55.33; | H, 4.94; | N, 12.10; | S, 9.23 |
| Found: | C, 55.36; | H, 4.95; | N, 12.23; | S, 9.11 |

Example 5

1,2,3,4-Tetrahydro-6-methyl-4-(3-nitrophenyl)-3-(2-propenyl)-2-thioxo-5-pyrimidinecarboxylic acid, ethyl ester

A) 1,4-Dihydro-2-[[(4-methoxyphenyl)methyl]thio]-6-methyl-4-(3-nitrophenyl)-5-pyrimidinecarboxylic acid, ethyl ester

A mixture of 13.58 g of 2-(3-nitrophenyl)methylene]-3-oxobutanoic acid, ethyl ester, 12.0 g of S-[(4-methoxyphenyl)methyl]thiopseudourea, hydrochloride and 4.18 g (0.051 mole) of sodium acetate in 90 ml of dimethylformamide was stirred and heated at 70°C for 4 hours. After cooling, ether was added followed by washing with water, sodium bicarbonate and brine. The dried solution was evaporated to give an oil which was treated with isopropyl ether to form 18.8 g of a cream colored solid, melting point 95—97°C.

B) 1,6-Dihydro-2-[[(4-methoxyphenyl)methyl]thio]-4-methyl-6-(3-nitrophenyl)-1-(2-propenyl)-5-pyrimidinecarboxylic acid, ethyl ester

A stirred suspension of 0.26 g (0.0054 mmole) of sodium hydride (50%) in 15 ml of tetrahydrofuran (0—5°C) was treated slowly with a solution of 2.0 g of 1,6 - dihydro - 2 - [[(4 - methoxyphenyl)-methyl]thio] - 4 - methyl - 6 - (3 - nitrophenyl) - 1 - (2 - propenyl) - 5 - pyrimidinecarboxylic acid, ethyl ester in 15 ml of tetrahydrofuran. After stirring for 10 minutes, 0.73 g (0.0060 mole) of allyl bromide was added and the mixture was stirred at room temperature overnight.

Ethyl acetate was added and the mixture was washed with 1N hydrochloric acid, sodium bicarbonate and brine. The dried solution was evaporated to give 2.3 g of an oil. Flash chromatography using dichloromethane gave 1.4 g of a yellow oil.

C) 1,2,3,4-Tetrahydro-6-methyl-4-(3-nitrophenyl)-3-(2-propenyl)-2-thioxo-5-pyrimidinecarboxylic acid, ethyl ester

A solution of 1.3 g (0.0027 mole) of 1,6 - dihydro - 2 - [[(4 - methoxyphenyl)methyl]thio] - 4 - methyl - 6 - (3 - nitrophenyl) - 1 - (2 - propenyl) - 5 - pyrimidinecarboxylic acid, ethyl ester in 20 ml of dichloromethane was treated with 1.0 ml (0.0130 mole) of trifluoroacetic acid and 0.4 g (0.0061 mole) of ethanethiol. After stirring overnight, the solvent was evaporated and the solid residue was triturated with ether to give 0.80 g of a cream colored solid, melting point 144—146°C. Flash chromatography using ethyl acetate/hexane (1:3) gave 0.42 g of the title compound, melting point 154—156°C.

| | | | | |
|---|---|---|---|---|
| Analysis Calc'd. for $C_{17}H_{19}N_3O_4S$: | C, 56.49; | H, 5.29; | N, 11.62; | S, 8.87 |
| Found: | C, 56.27; | H, 5.31; | N, 11.73; | S, 8.85 |

Example 6

1,2,3,4-Tetrahydro-6-methyl-3-(methylsulfonyl)-4-(3-nitrophenyl)-2-thioxo-5-pyrimidinecarboxylic acid, methyl ester

A) 1,6-Dihydro-2-[[(4-methoxyphenyl)methyl]thio]-4-methyl-1-(methylsulfonyl)-6-(3-nitrophenyl)-5-pyrimidinecarboxylic acid, methyl ester

A solution of 1,4 - dihydro - 2 - [[(4 - methoxyphenyl)methyl]thio] - 6 - methyl - 4 - (3 - nitrophenyl) - 5 - pyrimidinecarboxylic acid, methyl ester (1.14 g, 2.66 mmole; see Example 4B) in 15 ml of dichloromethane under argon at 0—5°C was treated with pyridine (0.42 ml, 0.42 g, 5.32 mmole) and methanesulfonyl chloride (0.28 ml, 0.41 g, 3.57 mmole). The mixture was then allowed to stir at room temperature overnight.

Volatiles were evaporated *in vacuo* and the residue, dissolved in ethyl acetate, was washed with 1N hydrochloric acid (twice), water (three times), sodium bicarbonate, water and saturated brine. The aqueous fractions were back extracted with fresh ethyl acetate. The combined organic fractions were dried (magnesium sulfate) and concentrated *in vacuo* to give 1.36 g of an oil. Flash chromatography on 250 ml of LPS-1 silica gel and elution with 2 liters of acetone/hexane (1:4) gave 0.58 g of product.

B) 1,2,3,4-Tetrahydro-6-methyl-3-(methylsulfonyl)-4-(3-nitrophenyl)-2-thioxo-5-pyrimidinecarboxylic acid, methyl ester

A solution of 1,6 - dihydro - 2 - [[(4 - methoxyphenyl)methyl]thio] - 4 - methyl - 1 - (methylsulfonyl) - 6 - (3 - nitrophenyl) - 5 - pyrimidinecarboxylic acid, methyl ester (0.57 g, 1.1 mmole) in 8 ml of dichloromethane under argon at room temperature was treated with trifluoroacetic acid (0.3 ml, 0.42 g, 3.8 mmole) and ethanethiol (0.2 ml, 0.16 g, 2.7 mmole) and allowed to react for 1 hour.

Volatiles were evaporated *in vacuo* and the residue was triturated with isopropyl ether overnight to give 320 mg of product, melting point 162—166°C. This was combined with an additional 70 mg of material from a second crop and recrystallized from methanol to give 310 mg of the title product, melting point 188—190°C.

| | | | | |
|---|---|---|---|---|
| Analysis Calc'd. for $C_{14}H_{15}N_3O_6S_2$: | C, 43.62; | H, 3.92; | N, 10.90; | S, 16.64 |
| Found: | C, 43.94; | H, 3.94; | N, 10.84; | S, 16.65 |

Example 7

1,2,3,4-Tetrahydro-6-methyl-4-(3-nitrophenyl)-3-(phenylsulfonyl)-2-thioxo-5-pyrimidinecarboxylic acid, ethyl ester

A) 1,6-Dihydro-2-[[(4-methoxyphenyl)methyl]thio]-4-methyl-6-(3-nitrophenyl)-1-(phenylsulfonyl)-5-pyrimidinecarboxylic acid, ethyl ester

A stirred solution of 1.5 g (0.0034 mole) of 1,4 - dihydro - 2 - [[(4 - methoxyphenyl)methyl]thio] - 6 - methyl - 4 - (3 - nitrophenyl) - 5 - pyrimidinecarboxylic acid, ethyl ester (see Example 5A) in 10 ml of dichloromethane containing 0.6 ml (0.0074 mole) of pyridine was treated gradually with a solution of 0.72 g (0.0041 mmole) of benzenesulfonyl chloride in 5 ml of dichloromethane. After 16 hours, dichloromethane was added and the solution was washed with water, 1N hydrochloric acid, sodium bicarbonate and brine. The dried solution was evaporated to give 2.1 g of an oil which slowly solidified. Treatment with ethyl acetate gave 0.48 g of a colorless solid, melting point 194—196°C (hydrochloric acid salt of 1,4 - dihydro - 2 - [[(4 - methoxyphenyl)methyl]thio] - 6 - methyl - 4 - (3 - nitrophenyl) - 5 - pyrimidinecarboxylic acid, ethyl ester.

The ethyl acetate solution was concentrated and flash chromatographed using ethyl acetate/hexane (1:3) to give 1.02 g of the title compound as an oil which slowly solidified, melting point 86—88°C.

| | | | |
|---|---|---|---|
| Analysis Calc'd. for $C_{28}H_{27}N_3O_7S_2$: | C, 57.81; | H, 4.67; | N, 7.22 |
| Found: | C, 57.91; | H, 4.92; | N, 7.03 |

B) 1,2,3,4-Tetrahydro-6-methyl-4-(3-nitrophenyl)-3-(phenylsulfonyl)-2-thioxo-5-pyrimidinecarboxylic acid, ethyl ester

A solution of 0.95 g (0.0016 mole) of 1,6 - dihydro - 2 - [[(4 - methoxyphenyl)methyl]thio] - 4 - methyl - 6 - (3 - nitrophenyl) - 1 - (phenylsulfonyl) - 5 - pyrimidinecarboxylic acid, ethyl ester, 0.6 ml (0.0066 mole) of trifluoroacetic acid and 0.24 g (0.0037 mole) of ethanethiol in 20 ml of dichloromethane was stirred at room temperature overnight. The solvent was evaporated and the residue (solid) was treated with isopropyl ether to give 0.68 g of the title compound as a colorless solid, melting point 162—164°C.

| | | | | |
|---|---|---|---|---|
| Analysis Calc'd. for $C_{20}H_{19}N_3O_6S_2$: | C, 52.04; | H, 4.14; | N, 9.10; | S, 13.89 |
| Found: | C, 51.77; | H, 4.09; | N, 8.96; | S, 13.71. |

Example 8

3-(Butylsulfonyl)-1,2,3,4-tetrahydro-6-methyl-4-(3-nitrophenyl)-2-thioxo-5-pyrimidinecarboxylic acid, ethyl ester

A) 1-(Butylsulfonyl)-1,6-dihydro-2-[[(4-methoxyphenyl)methyl]thio]-4-methyl-6-(3-nitrophenyl)-5-pyrimidinecarboxylic acid, ethyl ester

A solution of 2.0 g (0.0045 mole) of 1,4 - dihydro - 2 - [[(4 - methoxyphenyl)methyl]thio] - 6 - methyl - 4 - (3 - nitrophenyl) - 5 - pyrimidinecarboxylic acid, ethyl ester (see Example 5A) in 15 ml of dichloromethane containing 0.8 ml (0.0098 mole) of pyridine was cooled to −10°C and treated slowly with a solution of 0.85 g (0.0054 mole) of 1-butanesulfonyl chloride in 5 ml of dichloromethane. The ice bath was removed after 2 hours and the reaction was stirred at room temperature for 48 hours.

The hydrochloric acid salt of 1,4 - dihydro - 2 - [[(4 - methoxyphenyl)methyl]thio - 6 - methyl - 4 - (3 - nitrophenyl) - 5 - pyrimidinecarboxylic acid, ethyl ester (1.12 g of colorless solid, melting point 190—192°C) was separated and the solvent was evaporated to an oil. In ethyl acetate, this material was washed with water, 1N hydrochloric acid, sodium bicarbonate and brine. The dried solution was evaporated to give 1.2 g of an oil. Flash chromatography using ethyl acetate/hexane (1:4) gave 0.8 g of an oil which slowly solidified, melting point 66—68°C.

| | | | |
|---|---|---|---|
| Analysis Calc'd. for $C_{26}H_{31}N_3O_7S_2$: | C, 55.59; | H, 5.56; | N, 7.48 |
| Found: | C, 56.33; | H, 5.67; | N, 7.07 |

B) 3-(Butylsulfonyl)-1,2,3,4-tetrahydro-6-methyl-4-(3-nitrophenyl)-2-thioxo-5-pyrimidinecarboxylic acid, ethyl ester

A solution of 0.80 g (0.0014 mole) of 1 - (Butylsulfonyl) - 1,6 - dihydro - 2 - [[(4 - methoxyphenyl)-methyl]thio - 4 - methyl - 6 - (3 - nitrophenyl) - 5 - pyrimidinecarboxylic acid, ethyl ester, 0.52 ml (0.0057 mole) of trifluoroacetic acid and 0.21 g (0.0032 mole) of ethanethiol in 15 ml of dichloromethane was stirred at room temperature for 24 hours. The solvent was evaporated, and the residue was flash chromatographed using ethyl acetate/hexane (1:4) to give an oil which solidified very slowly. Trituration with isopropyl ether gave 0.33 g of the title compound as a colorless solid, melting point 118—120°C.

| | | | | |
|---|---|---|---|---|
| Analysis Calc'd. for $C_{18}H_{23}N_3O_6S_2$: | C, 48.96; | H, 5.25; | N, 9.51; | S, 14.52 |
| Found: | C, 48.99; | H, 5.42; | N, 9.32; | S, 14.48 |

Example 9

1,2,3,4-Tetrahydro-6-methyl-3-(methylsulfonyl)-4-(3-nitrophenyl)-2-thioxo-5-pyrimidinecarboxylic acid, ethyl ester

A) 1,6-Dihydro-2-[[(4-methoxyphenyl)methyl]thio]-4-methyl-1-(methylsulfonyl)-6-(3-nitrophenyl)-5-pyrimidinecarboxylic acid, ethyl ester

A solution of 2.0 g (0.0045 mole) of 1,4 - dihydro - 2 - [[(4 - methoxyphenyl)methyl]thio] - 6 - methyl - 4 - (3 - nitrophenyl) - 5 - pyrimidinecarboxylic acid, ethyl ester (see example 5A) in 15 ml of dichloromethane containing 0.71 g (0.0090 mole) of pyridine was cooled to −10°C and treated slowly with a solution of 0.62 g (0.0054 mole) of methanesulfonyl chloride in 5 ml of dichloromethane. After stirring at

room temperature overnight, a small amount of the hydrochloric acid salt of 1,4 - dihydro - 2 - [[(4 - methoxyphenyl)methyl]thio] - 6 - methyl - 4 - (3 - nitrophenyl) - 5 - pyrimidinecarboxylic acid, ethyl ester had precipitated. After filtration, additional dichloromethane was added and the solution was washed with water, 1N hydrochloric acid, sodium bicarbonate and brine. The dried solution was evaporated and the residue was flash chromatographed using dichloromethane to give 2.0 g of an oil.

B) 1,2,3,4-Tetrahydro-6-methyl-3-(methylsulfonyl)-4-(3-nitrophenyl)-2-thioxo-5-pyrimidinecarboxylic acid, ethyl ester

A solution of 2.0 g (0.0038 mole) of 1,6 - dihydro - 2 - [[(4 - methoxyphenyl)methyl]thio] - 4 - methyl - 1 - (methylsulfonyl) - 6 - (3 - nitrophenyl) - 5 - pyrimidinecarboxylic acid, ethyl ester, 1.4 ml (0.0153 mole) of trifluoroacetic acid and 0.57 g (0.0086 mole) of ethanethiol in 20 ml of dichloromethane was stirred at room temperature for 40 hours and heated to reflux for 8 hours. The mixture was cooled and filtered to give 1.05 g of the compound as a colorless solid, melting point 161—163°C.

Analysis Calc'd. for $C_{15}H_{17}N_3O_6S_2$: C, 45.10; H, 4.28; N, 10.51; S, 16.05
Found: C, 45.03; H, 4.17; N, 10.42; S, 16.04

Example 10

1,2,3,6-Tetrahydro-4-methyl-1-(methylsulfonyl)-5-(3-nitrophenyl)-2-oxo-5-pyrimidinecarboxylic acid, ethyl ester

A) 1,4-Dihydro-2-methoxy-6-methyl--4-(3-nitrophenyl)-5-pyrmidinecarboxylic acid, ethyl ester

A reaction containing 2-[(3-nitrophenyl)methylene]-3-oxobutanoic acid, ethyl ester (2.62 g, 10.0 mmole), O-methylpseudourea hydrogen sulfate (1.72 g, 10.0 mmole), and sodium bicarbonate (2.52 g, 30.0 mmole) in dimethylformamide (7 ml) was heated at 65—70°C for 16 hours. The reaction mixture was allowed to cool to room temperature, diluted with ethyl acetate, and filtered. The filtrate was washed with water and brine, and then dried over anhydrous magnesium sulfate. Evaporation of the solvent gave a yellow oil which was purified by flash chromatography (5% ethyl acetate in dichloromethane). The resulting foam was crystallized from isopropyl ether/hexanes to provide 2.41 g of 1,4-dihydro-2-methoxy-6-methyl-4-(3-nitrophenyl)-5-pyrimidinecarboxylic acid, ethyl ester as a colorless crystalline product; melting point 103.5—105°C.

Analysis Calc'd. for $C_{15}H_{17}N_3O_5$: C, 56.42; H, 5.37; N, 13.16
Found: C, 56.52; H, 5.35; N, 13.03

B) 1,2,3,6-Tetrahydro-4-methyl-1-(methylsulfonyl)-5-(3-nitrophenyl)-2-oxo-5-pyrimidinecarboxylic acid, ethyl ester

A solution of 1,4-dihydro-2-methoxy-6-methyl-4-(3-nitrophenyl)-5-pyrimidinecarboxylic acid, ethyl ester (2.00 g, 6.26 mmol), pyridine (2.5 ml, 31 mmol), and 4-dimethylaminopyridine (36 mg, 0.3 mmol) in distilled dichloromethane in an ice bath under argon was treated via syringe with distilled methanesulfonyl chloride (0.63 ml, 8.14 mmol). After five minutes, the ice bath was removed, and the reaction was stirred at room temperature overnight. The mixture was then evaporated. The residue was taken up in tetrahydrofuran (10 ml) and methanol (20 ml) and the resulting suspension was treated with 1N hydrochloric acid (8 ml) and 5N hydrochloric acid (2 ml). After stirring for 2.0 hours at room temperature, the reaction was quenched with sodium bicarbonate and extractd with ethyl acetate. The organic phase was then washed with saturated sodium chloride. Flash chromatography (ethyl acetate/hexanes (1:1)) and crystallization from dichloromethane/isopropyl ether gave the title compound as white crystals (605 mg), melting point 175—176°C.

Analysis Calc'd. for $C_{15}H_{17}N_3O_7S$: C, 46.99; H, 4.47; N, 10.96; S, 8.36
Found: C, 47.12; H, 4.39; N, 10.55; S, 8.17

Example 11

1,2,3,6-Tetrahydro-4-methyl-6-(3-nitrophenyl)-2-oxo-1-(phenylsulfonyl)-5-pyrimidinecarboxylic acid, ethyl ester

A) 1,2,3,6-Tetrahydro-4-methyl-6-(3-nitrophenyl)-2-methoxy-1-(phenylsulfonyl)-5-pyrimidinecarboxylic acid, ethyl ester

A solution of 1,5-dihydro-2-methoxy-6-methyl-4-(3-nitrophenyl)-5-pyrimidinecarboxylic acid, ethyl ester (3.19 g, 10.0 mmole; see Example 10A) and distilled triethylamine (4.18 ml, 30.0 mmol) in distilled dichloromethane (20 ml) in an ice bath under argon was treated dropwise via syringe with benzenesulfonyl chloride (1.53 ml, 12.0 mmol). The reaction was then stirred at room temperature overnight; and then over the weekend. The mixture was partitioned between ethyl acetate and water. The organic phase was washed with saturated sodium chloride and flash chromatographed to give the title compound as a light brown oil (2.94 g).

B) 1,2,3,6-Tetrahydro-4-methyl-6-(3-nitrophenyl)-2-oxo-1-(phenylsulfonyl)-5-pyrimidinecarboxylic acid, ethyl ester

A solution of 1,2,3,6 - tetrahydro - 4 - methyl - 6 - (3 - nitrophenyl) - 2 - methoxy - 1 - (phenylsulfonyl) - 5 - pyrimidinecarboxylic acid, ethyl ester (1.49 g, 3.24 mmol) in tetrahydrofuran-methanol (20

ml each) was treated with 5N hydrochloric acid (5.0 ml) and stirred at room temperature overnight. The reaction mixture was evaporated and partitioned between ethyl acetate and water. The organic phase was washed with saturated sodium bicarbonate and saturated sodium chloride. Flash chromatography (acetone/hexanes (1:5)) and crystallization from dichloromethane/isopropyl ether gave the title compound as white crystals (589 mg), melting point 187—188°C.

| | | | | |
|---|---|---|---|---|
| Analysis Calc'd. for $C_{20}H_{19}N_3O_7S$: | C, 53.93; | H, 4.30; | N, 9.43; | S, 7.20 |
| Found: | C, 53.71; | H, 4.19; | N, 9.27; | S, 7.13 |

Additional compounds falling within the scope of this invention are:

4-(2,3-dichlorophenyl)-1,2,3,4-tetrahydro-6-methyl-3-[3-[(methyl)(phenylmethyl)amino]propyl]-2-oxo-5-pyrimidinecarboxylic acid, ethyl ester

1,2,3,4-tetrahydro-6-methyl-4-(2-nitrophenyl)-2-oxo-3-propyl-5-pyrimidinecarboxylic acid, 1-phenylmethyl-4-piperidinyl ester

1,2,3,4-tetrahydro-3,6-dimethyl-4-(3-nitrophenyl)-2-oxo-5-pyrimidinecarboxylic acid, 1-methylethyl ester

1,2,3,4-tetrahydro-6-methyl-3-[4-(dimethylamino)butyl]-2-oxo-4-[2-(trifluoromethyl)phenyl]-5-pyrimidinecarboxylic acid, ethyl ester

4-(7-benzofurazanyl)-1,2,3,4-tetrahydro-6-methyl-2-oxo-3-(2-propenyl)-5-pyrimidinecarboxylic acid, 2-[(methyl)(phenylmethyl)amino]ethyl ester

1,2,3,4-tetrahydro-6-methyl-4-[2-(methylthio)-3-pyridinyl]-2-oxo-3-[4-(4-pyrimidinyl)butyl]-5-pyrimidinecarboxylic acid, ethyl ester

4-(2-chloro-3-nitrophenyl)-1,2,3,4-tetrahydro-6-methyl-2-oxo-3-[3-[4-(phenylmethyl)-1-piperazinyl]propyl]-5-pyrimidinecarboxylic acid, ethyl ester

1,2,3,4-tetrahydro-6-methyl-4-(3-nitrophenyl)-2-oxo-3-[2-[4-(diphenylmethyl)-1-piperazinyl]ethyl]-5-pyrimidinecarboxylic acid, ethyl ester

4-(2-chlorophenyl)-1,2,3,4-tetrahydro-6-methyl-2-oxo-3-(2-propenyl)-5-pyrimidinecarboxylic acid, 2-[4-(phenylmethyl)-1-piperazinyl]ethyl ester

1,2,3,4-tetrahydro-6-methyl-4-(3-nitrophenyl)-2-oxo-3-(3-phenylpropyl)-5-pyrimidinecarboxylic acid, 2-[4-(diphenylmethyl)-1-piperazinyl]ethyl ester

4-(2,3-dichlorophenyl)-1,2,3,4-tetrahydro-6-methyl-3-[3-[(methyl)(phenylmethyl)amino]propyl]-2-thioxo-5-pyrimidinecarboxylic acid, ethyl ester

1,2,3,4-tetrahydro-6-methyl-4-(2-nitrophenyl)-3-propyl-2-thioxo-5-pyrimidinecarboxylic acid, 1-phenylmethyl-4-piperidinyl ester

1,2,3,4-tetrahydro-3,6-dimethyl-4-(3-nitrophenyl)-2-thioxo-5-pyrimidinecarboxylic acid, 1-methylethyl ester

1,2,3,4-tetrahydro-6-methyl-3-[4-(dimethylamino)butyl]-2-thioxo-4-[2-(trifluoromethyl)phenyl]-5-pyrimidinecarboxylic acid, ethyl ester

4-(7-benzofurazanyl)-1,2,3,4-tetrahydro-6-methyl-3-(2-propenyl)-2-thioxo-5-pyrimidinecarboxylic acid, 2-[(methyl)(phenylmethyl)amino]ethyl ester

1,2,3,4-tetrahydro-6-methyl-4-[2-(methylthio)-3-pyridinyl]-3-[4-(4-pyridinyl)butyl]-2-thioxo-5-pyrimidinecarboxylic acid, ethyl ester

4-(2-chloro-3-nitrophenyl)-1,2,3,4-tetrahydro-6-methyl-3-[3-[4-(phenylmethyl)-1-piperazinyl]propyl]-2-thioxo-5-pyrimidinecarboxylic acid, ethyl ester

1,2,3,4-tetrahydro-6-methyl-4-(3-nitrophenyl)-3-[2-[4-(diphenylmethyl)-1-piperazinyl]ethyl]-2-thioxo-5-pyrimidinecarboxylic acid, ethyl ester

4-(2-chlorophenyl)-1,2,3,4-tetrahydro-6-methyl-3-(2-propenyl)-2-thioxo-5-pyrimidinecarboxylic acid, 2-[4-(phenylmethyl)-1-piperazinyl]ethyl ester

1,2,3,4-tetrahydro-6-methyl-4-(3-nitrophenyl)-3-(3-phenylpropyl)-2-thioxo-5-pyrimidinecarboxylic acid, 2-[4-(diphenylmethyl)-1-piperazinyl]ethyl ester

6-(2,3-dichlorophenyl)-1,2,3,4-tetrahydro-4-methyl-2-oxo-1-(phenylsulfonyl)-5-pyrimidinecarboxylic acid, ethyl ester

1-(1-butylsulfonyl)-1,2,3,4-tetrahydro-4-methyl-6-(3-nitrophenyl)-2-oxo-5-pyrimidinecarboxylic acid, 2-(dimethylamino)ethyl ester

1,2,3,4-tetrahydro-4-methyl-1-(methylsulfonyl)-6-(2-nitrophenyl)-2-oxo-5-pyrimidinecarboxylic acid, ethyl ester

1,2,3,4-tetrahydro-4-methyl-1-[3-[(phenylmethyl)(methyl)amino]propyl]sulfonyl]-6-(3-nitrophenyl)-2-oxo-5-pyrimidinecarboxylic acid, 1-methylethyl ester

1,2,3,4-tetrahydro-4-methyl-1-[[3-[4-(phenylmethyl)-1-piperazinyl]propyl]sulfonyl]-6-(3-nitro-phenyl)-2-oxo-5-pyrimidinecarboxylic acid, ethyl ester

1,2,3,4-tetrahydro-4-methyl-1-[[3-(dimethylamino)propyl]sulfonyl]-2-oxo-6-[2-(trifluoromethyl)-phenyl]-5-pyrimidinecarboxylic acid, ethyl ester

1,2,3,4-tetrahydro-4-methyl-2-oxo-1-[(phenylmethyl)sulfonyl]-6-[2-(methylthio)-3-pyridyl]-5-pyrimidinecarboxylic acid, ethyl ester

6-(4-benzoxadiazolyl)-1,2,3,4-tetrahydro-4-methyl-2-oxo-1-(phenylsulfonyl)-5-pyrimidinecarboxylic acid, 2-[(phenylmethyl)(methyl)amino]ethyl ester

1-(1-butylsulfonyl)-6-(2-chloro-3-nitrophenyl)-1,2,3,4-tetrahydro-4-methyl-2-oxo-5-pyrimidinecarboxylic acid, 1-(phenylmethyl)-4-piperidinyl ester

6-(2,3-dichlorophenyl)-1,2,3,4-tetrahydro-4-methyl-1-(phenylsulfonyl)-2-thioxo-5-pyrimidinecarboxylic acid, ethyl ester

1-(1-butylsulfonyl)-1,2,3,4-tetrahydro-4-methyl-6-(3-nitrophenyl)-2-thioxo-5-pyrimidinecarboxylic acid, 2-(dimethylamino)ethyl ester

1,2,3,4-tetrahydro-4-methyl-1-(methylsulfonyl)-6-(2-nitrophenyl)-2-thioxo-5-pyrimidinecarboxylic acid, ethyl ester

1,2,3,4-tetrahydro-4-methyl-1-[3-[(phenylmethyl)(methyl)amino]propyl]sulfonyl]-6-(3-nitrophenyl)-2-thioxo-5-pyrimidinecarboxylic acid, 1-methylethyl ester

1,2,3,4-tetrahydro-4-methyl-1-[[3-[4-(phenylmethyl)-1-piperazinyl]propyl]sulfonyl]-6-(3-nitrophenyl)2-thioxo-5-pyrimidinecarboxylic acid, ethyl ester

1,2,3,4-tetrahydro-4-methyl-1-[[3-(dimethylamino)propyl]sulfonyl]-2-thioxo-6-(trifluoromethyl)-phenyl]-5-pyrimidinecarboxylic acid, ethyl ester

1,2,3,4-tetrahydro-4-methyl-2-thioxo-1-[(phenylmethyl)sulfonyl]-6-[2-(methylthio)-3-pyridyl]-5-pyrimidinecarboxylic acid, ethyl ester

6-(4-benzoxadiazolyl)-1,2,3,4-tetrahydro-4-methyl-2-thioxo-1-(phenylsulfonyl)-5-pyrimidinecarboxylic acid, 2-[(phenylmethyl)(methyl)amino], ethyl ester

1-(1-butylsulfonyl)-6-(2-chloro-3-nitrophenyl)-1,2,3,4-tetrahydro-4-methyl-2-thioxo-5-pyrimidinecarboxylic acid, 1-(phenylmethyl)-4-piperidinyl ester

**Claims**

1. A compound having the formula

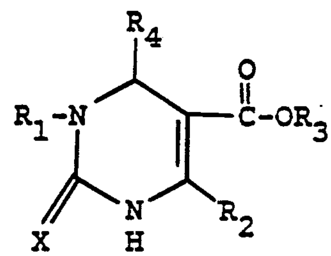,

or a pharmaceutically acceptable salt thereof wherein

X is oxygen or sulfur;

$R_1$ is alkyl, cycloalkyl, alkenyl, alkynyl, aryl, $-(CH_2)_n-Y_2$, $-(CH_2)_p-Y_3$, halo substituted alkyl, or

$$-\underset{\underset{O}{\|}}{\overset{\overset{O}{\|}}{S}}-Y_4;$$

$R_2$ is hydrogen, alkyl, alkenyl, alkynyl, cycloalkyl, aryl, $-(CH_2)_n-Y_1$, or halo substituted alkyl;

$R_3$ is hydrogen, alkyl, cycloalkyl, aryl, heterocyclo, $-(CH_2)_n-Y_2$, $-(CH_2)_p-Y_3$, or halo substituted alkyl;

$R_4$ is aryl or heterocyclo;

$Y_1$ is cycloalkyl, aryl, heterocyclo, hydroxyl, alkoxy, aryl-$(CH_2)_m-O-$, mercapto, alkylthio, aryl$-(CH_2)_m-S-$, amino, substituted amino, carbamoyl,

$$\text{(substituted amino)-}\overset{\overset{O}{\|}}{C}-,\quad \text{heterocyclo-}(CH_2)_m-\overset{\overset{O}{\|}}{C}-,$$

carboxyl, alkoxycarbonyl,

$$\text{alkyl-}\overset{\overset{O}{\|}}{C}-,\quad \text{aryl-}(CH_2)_m-\overset{\overset{O}{\|}}{C}-,\quad \text{alkyl-}\overset{\overset{O}{\|}}{C}-O-\ \text{or}\ \text{aryl-}(CH_2)_m-\overset{\overset{O}{\|}}{C}-O-;$$

$Y_2$ is cycloalkyl, aryl, heterocyclo, carbamoyl,

$$\text{(substituted amino)-}\overset{\text{O}}{\overset{\|}{\text{C}}}\text{—,}$$

carboxyl, alkoxycarbonyl,

$$\text{alkyl-}\overset{\text{O}}{\overset{\|}{\text{C}}}\text{—, aryl-(CH}_2\text{)}_m\text{—}\overset{\text{O}}{\overset{\|}{\text{C}}}\text{— or heterocyclo-(CH}_2\text{)}_m\text{—}\overset{\text{O}}{\overset{\|}{\text{C}}}\text{—;}$$

$Y_3$ is hydroxyl, alkoxy, aryl-$(CH_2)_m$—O—, mercapto, alkylthio, aryl-$(CH_2)_m$—S—,

$$\text{alkyl-}\overset{\text{O}}{\overset{\|}{\text{C}}}\text{—O—, aryl-(CH}_2\text{)}_m\text{—}\overset{\text{O}}{\overset{\|}{\text{C}}}\text{—O—,}$$

amino, or substituted amino;

$Y_4$ is alkyl, cycloalkyl, aryl, heterocyclo, —$(CH_2)_n$—$Y_1$ or halo substituted alkyl;

the terms "alkyl" and "alkoxy" refer to straight and branched chain groups having 1 to 8 carbon atoms;

the term "halo substituted alkyl" refers to alkyl groups in which one or more hydrogen atoms have been replaced by chloro, bromo or fluoro groups;

the term "aryl" refers to phenyl and phenyl substituted with 1, 2 or 3 alkyl, alkoxy, alkylthio, halo, nitro, cyano, hydroxy, amino, alkylamino, dialkylamino, trifluoromethyl, isothiocyanato, isocyanato, or difluoromethoxy groups;

the terms "alkenyl" and "alkynyl" refer to straight and branched chain groups having 2 to 8 carbon atoms;

the term "cycloalkyl" refers to cycloalkyl groups having 3, 4, 5, 6 or 7 carbon atoms.

the term "heterocyclo" refers to fully saturated or unsaturated rings of 5 or 6 atoms having 1 to 4 nitrogen atoms and/or 1 or 2 oxygen or sulfur atoms, provided that the total number of heteroatoms is 4 or less; wherein the term "heterocyclo" also refers to bicyclic rings wherein the 5- or 6-membered ring is fused to a benzene ring and the bicyclic ring is attached by way of a carbon atom in the benzene ring and wherein each ring comprises substituents selected from alkyl, arylalkyl, diarylalkyl, alkylthio, alkoxy, halo, nitro, oxo, cyano, hydroxy, amino, alkylamino, dialkylamino, trifluoromethyl, isocyanato, isothiocyanato, and difluoromethoxy;

the term "substituted amino" refers to a group of the formula —$NZ_1Z_2$ wherein $Z_1$ is hydrogen, alkyl, or aryl-$(CH_2)_m$— and $Z_2$ is alkyl or aryl-$(CH_2)_m$—; or $Z_1$ and $Z_2$, taken together with the nitrogen to which they are attached, are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, 1-pyrrolidinyl, 1-piperidinyl, or 1-azepinyl substituted with alkyl, alkoxy, alkylthio, halo, trifluoromethyl or hydroxy.

m is 0 or an integer of 1 to 6;

n is an integer of 1 to 6; and

p is an integer of 2 to 6.

2. A compound in accordance with claim 1 wherein X is oxygen.

3. A compound in accordance with claim 1 wherein X is sulfur.

4. A compound in accordance with claim 1 wherein $R_2$ is methyl.

5. A compound in accordance with claim 1 wherein $R_3$ is alkyl.

6. A compound in accordance with claim 1 wherein $R_4$ is 3-nitrophenyl.

7. A compound in accordance with claim 1 wherein $R_1$ is alkyl.

8. A compound in accordance with claim 1 wherein $R_1$ is alkenyl.

9. A compound in accordance with claim 1 wherein $R_1$ is alkynyl.

10. A compound in accordance with claim 1 wherein $R_1$ is aryl.

11. A compound in accordance with claim 1 wherein $R_1$ is arylalkyl.

12. A compound in accordance with claim 1 wherein $R_1$ is —$(CH_2)_n$—$Y_2$ or —$(CH_2)_p$—$Y_3$.

13. A compound in accordance with claim 1 wherein $R_1$ is

$$\text{—}\underset{\text{O}}{\underset{\|}{\overset{\text{O}}{\overset{\|}{\text{S}}}}}\text{—Y}_4\text{.}$$

14. A compound in accordance with claim 1 wherein X is sulfur, $R_2$ is sulfur, $R_2$ is methyl, $R_3$ is alkyl and $R_4$ is 3-nitrophenyl.

15. A compound in accordance to claim 1 for use in lowering blood pressure in a mammalian host in need thereof.

16. A compound in accordance to claim 1 for use as a calcium entry blocking vasodilator in a mammalian host in need thereof.

**Patentansprüche**

1. Verbindung der Formel

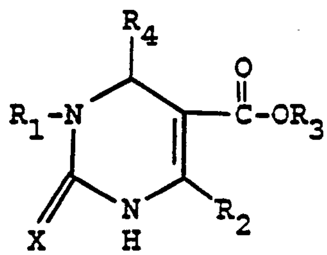

,

oder ein pharmazeutisch verträgliches Salz davon, wobei X ein Sauerstoff- oder Schwefelatom ist;

$R_1$ ein Alkyl-, Cycloalkyl-, Alkenyl-, Alkinyl- oder Arylrest, $-(CH_2)_n-Y_2$, $-(CH_2)_p-Y_3$, ein halogensubstituierter Alkylrest oder

$$-\overset{\overset{O}{\|}}{\underset{\underset{O}{\|}}{S}}-Y_4 \text{ ist;}$$

$R_2$ ein Wasserstoffatom oder ein Alkyl-, Alkenyl-, Alkinyl-, Cycloalkyl- oder Arylrest, $-(CH_2)_n-Y_1$ oder halogensubstituierter Alkylrest ist;

$R_3$ ein Wasserstoffatom oder ein Alkyl-, Cycloalkyl-, Aryl- oder heterocyclischer Rest, $-(CH_2)_n-Y_2$, $-(CH_2)_p-Y_3$ oder ein halogensubstituierter Alkylrest ist;

$R_4$ ein Aryl- oder heterocyclischer Rest ist;

$Y_1$ ein Cycloalkyl-, Aryl-, heterocyclischer, Hydroxy-, Alkoxy-, Aryl-$(CH_2)_m-O-$, Mercapto-, Alkylthio-, Aryl-$(CH_2)_m-S-$, Amino-, substituierter Amino-, Carbamoyl,

$$\text{(substituierter Amino)-}\overset{\overset{O}{\|}}{C}-,\ \text{Heterocyclo-}(CH_2)_m-\overset{\overset{O}{\|}}{C}-,\ \text{Carboxy-, Alkoxycarbonyl, Alkyl-}\overset{\overset{O}{\|}}{C}-,$$

$$\text{Aryl-}(CH_2)_m-\overset{\overset{O}{\|}}{C}-,\ \text{Alkyl-}\overset{\overset{O}{\|}}{C}-O-\ \text{oder}\ \text{Aryl-}(CH_2)_m-\overset{\overset{O}{\|}}{C}-O-\text{Rest ist;}$$

$Y_2$ ein Cycloalkyl-, Aryl-, heterocyclischer, Carbamoyl-,

$$\text{(substituierter Amino)-}\overset{\overset{O}{\|}}{C}-,\ \text{Carboxy-, Alkoxycarbonyl-, Alkyl-}\overset{\overset{O}{\|}}{C}-,$$

$$\text{Aryl-}(CH_2)_m-\overset{\overset{O}{\|}}{C}-\ \text{oder}\ \text{Heterocyclo-}(CH_2)_m-\overset{\overset{O}{\|}}{C}-\text{Rest ist;}$$

$Y_3$ ein Hydroxy-, Alkoxy-, Aryl-$(CH_2)_m-O-$, Mercapto, Alkylthio, Aryl-$(CH_2)_m-S-$,

$$\text{Alkyl-}\overset{\overset{O}{\|}}{C}-O-,\ \text{Aryl-}(CH_2)_m-\overset{\overset{O}{\|}}{C}-O-,$$

Amino- oder substituierter Aminorest ist;

$Y_4$ ein Alkyl-, Cycloalkyl-, Aryl- oder heterocyclischer Rest, $-(CH_2)_n-Y_1$ oder ein halogensubstituierter Alkylrest ist;

die Ausdrücke "Alkylrest" und "Alkoxyrest" sich auf lineare und verzweigte Reste mit 1 bis 8 Kohlenstoffatomen beziehen;

der Ausdruck "halogensubstituierter Alkylrest" sich auf Alkylreste bezieht, in denen eines oder mehrere Wasserstoffatome durch Chlor-, Brom- oder Fluoratome ersetzt sind;

der Ausdruck "Arylrest" sich auf eine Phenylgruppe oder Phenylreste, substituiert mit 1, 2 oder 3 Alkyl-, Alkoxy-, Alkylthio-, Halogen-, Nitro-, Cyano-, Hydroxy-, Amino-, Akylamino-, Dialkylamino-, Trifluormethyl-, Isothiocyanato-, Isocyanato- oder Difluormethoxyresten, bezieht;

die Ausdrücke "Alkenylrest" und "Alkinylrest" sich auf lineare und verzweigte Reste mit 2 bis 8 Kohlenstoffatomen bezhiehen

der Ausdruck "Cycloalkylrest" sich auf Cycloalkylreste mit 3, 4, 5, 6 oder 7 Kohlenstoffatomen bezieht;

der Ausdruck "heterocyclisch" sich auf gesättigte oder ungesättigte Ringe von 5 oder 6 Atomen mit 1 bis 4 Stickstoffatomen und/oder 1 oder 2 Sauerstoff- oder Schwefelatomen bezieht, mit der Maßgabe, daß die Gesamtzahl der Heteroatome 4 oder weniger beträgt und der Ausdruck "heterocyclisch" sich auch auf bicyclische Ringe bezieht, in denen ein 5- oder 6-Ring mit einem Benzolring vereint ist, und der bicyclische Ring über ein Kohlenstoffatom im Benzolring gebunden ist, und wobei jeder Ring Substituenten aus der Gruppe Alkyl-, Arylalkyl-, Diarylalkyl-, Alkylthio-, Alkoxy-, Halogen-, Nitro-, Oxo-, Cyano-, Hydroxy-, Amino-, Alkylamino-, Dialkylamino-, Trifluormethyl-, Isocyanato-, Isothiocyanato- und Difluormethoxyreste aufweist;

der Ausdruck "substituierter Aminorest" sich auf einen Rest der Formel $-NZ_1Z_2$ bezieht, in der $Z_1$ ein Wasserstoffatom, ein Alkyl- oder Aryl-$(CH_2)_m$-Rest ist und $Z_2$ ein Alkyl- oder Aryl-$(CH_2)_m$-Rest ist, oder $Z_1$ und $Z_2$ zusammen mit dem Stickstoffatom, an das sie gebunden sind, einen 1-Pyrrolidinyl-, 1-Piperidinyl-, 1-Azepinyl-, 4-Morpholinyl-, 4-Thioamorpholinyl-, 1-Piperazinyl-, 4-Alkyl-1-piperazinyl-, 4-Arylalkyl-1-piperazinyl-, 4-Diarylalkyl-1-piperazinyl-, 1-Pyrrolidinyl-, 1-Piperidinyl- oder 1-Azepinylrest, substituiert mit einem Alkyl-, Alkoxy- oder Alkylthiorest, einem Halogenatom oder einem Trifluormethyl- oder Hydroxyrest bildet, ist;

m der Wert 0 oder eine ganze Zahl von 1 bis 6 ist;

n eine ganze Zahl von 1 bis 6 ist; und

p eine ganze Zahl von 2 bis 6 ist.

2. Verbindung nach Anspruch 1, in der X ein Sauerstoffatom ist.

3. Verbindung nach Anspruch 1, in der X ein Schwefelatom ist.

4. Verbindung nach Anspruch 1, in der $R_2$ eine Methylgruppe ist.

5. Verbindung nach Anspruch 1, in der $R_3$ ein Alkylrest ist.

6. Verbindung nach Anspruch 1, in der $R_4$ eine 3-Nitrophenylgruppe ist.

7. Verbindung nach Anspruch 1, in der $R_4$ ein Alkylrest ist.

8. Verbindung nach Anspruch 1, in der $R_1$ ein Alkenylrest ist.

9. Verbindung nach Anspruch 1, in der $R_1$ ein Alkinylrest ist.

10. Verbindung nach Anspruch 1, in der $R_1$ ein Arylrest ist.

11. Verbindung nach Anspruch 1, in der $R_1$ ein Arylalkylrest ist.

12. Verbindung nach Anspruch 1, in der $R_1$ ein Rest $-(CH_2)_n-Y_2$ oder $-(CH_2)_p-Y_3$-Rest ist.

13. Verbindung nach Anspruch 1, in der $R_1$ ein Rest

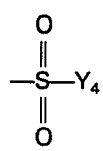 ist.

14. Verbindung nach Anspruch 1, in der X ein Schwefelatom, $R_2$ eine Methylgruppe, $R_3$ ein Alkylrest und $R_4$ eine 3-Nitrophenylgruppe sind.

15. Verbindung nach Anspruch 1 zur Verwendung zum Blutdrucksenken in einem Säuger, bei dem dies notwendig ist.

16. Verbindung nach Anspruch 1 zur Verwendung als den Calciumzutritt blockierender Vasodilator in einem Säuger, bei dem dies notwendig ist.

## Revendications

1. Composé de formule

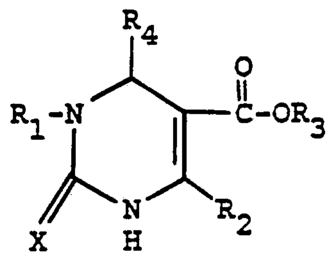

,

ou sel pharmaceutiquement acceptable de celui-ci, formule dans laquelle

X est l'oxygène ou le soufre;

$R_1$ est un radical alkyle, cycloalkyle, alcényle, alcynyle, aryle, $-(CH_2)_n-Y_2$, $-(CH_2)_p-Y_3$, alkyle halo-substitué ou

$$-\underset{\underset{O}{\|}}{\overset{\overset{O}{\|}}{S}}-Y_4;$$

$R_2$ est l'hydrogène ou un radical alkyle, alcényle, alcynyle, cycloalkyle, aryle, $-(CH_2)_n-Y_1$ ou alkyle halo-substitué;

$R_3$ est l'hydrogène ou un radical alkyle, cycloalkyle, aryle, hétérocyclique, $-(CH_2)_n-Y_2$, $-(CH_2)_p-Y_3$ ou alkyle halo-substitué;

$R_4$ est un radical aryle ou hétérocyclique;

$Y_1$ est un radical cycloalkyle, aryle, hétérocyclique, hydroxyle, alcoxy, aryl-$(CH_2)_m-O-$, mercapto, alkylthio, aryl-$(CH_2)_m-S-$, amine, amine substitué, carbamoyle,

$$\text{(amino substitué)-}\overset{\overset{O}{\|}}{C}-,\quad \text{hétérocyclo-}(CH_2)_m-\overset{\overset{O}{\|}}{C}\text{, carboxyle, alcoxycarbonyle,}$$

$$\text{alkyl-}\overset{\overset{O}{\|}}{C}-,\quad \text{aryl-}(CH_2)_m-\overset{\overset{O}{\|}}{C},\quad \text{alkyl-}\overset{\overset{O}{\|}}{C}-O-\quad \text{ou aryl-}(CH_2)_m-\overset{\overset{O}{\|}}{C}-O-;$$

$Y_2$ est un radical cycloalkyle, aryle, hétérocyclique, carbamoyle,

$$\text{(amino substitué)-}\overset{\overset{O}{\|}}{C}-,\quad \text{carboxyle, alcoxycarbonyle, alkyl-}\overset{\overset{O}{\|}}{C}-,$$

$$\text{aryl-}(CH_2)_m-\overset{\overset{O}{\|}}{C}\quad \text{ou hétérocyclo-}(CH_2)_m-\overset{\overset{O}{\|}}{C}-;$$

$Y_3$ est un radical hydroxyle, alcoxy, aryl-$(CH_2)_m-O-$, mercapto, alkylthio, aryl-$(CH_2)_m-S-$,

$$\text{alkyl-}\overset{\overset{O}{\|}}{C}-O-,\quad \text{aryl-}(CH_2)_m-\overset{\overset{O}{\|}}{C}-O-\text{, amine ou amine substitué;}$$

$Y_4$ est un radical alkyle, cycloalkyle, aryle, hétérocyclique, $-(CH_2)_n-Y_2$ ou alkyle halo-substitué;

les termes "alkyle" "alcoxy" désignent des radicaux à chaîne droite ou ramifiée de 1 à 8 atomes de carbone;

le terme "alkyle halo-substitué" désigne des radicaux alkyle dans lesquels ou un plusieurs atomes d'hydrogène ont été remplacés par des atomes de chlore, de brome ou de fluor;

le terme "aryle" désigne le radical phényle éventuellement substitué par 1, 2 ou 3 groupements ou

atomes alkyle, alcoxy, alkylthio, halogène, nitro, cyano, hydroxy, amine, alkylamine, dialkylamine, trifluorométhyle, isothiocyanate, isocyanate ou difluorométhoxy;

les termes "alcényle" et "alcynyle" désignent des radicaux à chaîne droite ou ramifié de 2 à 8 atomes de carbone;

le terme "cycloalkyle" désigne des radicaux cycloalkyle ayant 3, 4, 5, 6 ou 7 atomes de carbone;

le terme "hétérocycle" désigne des noyaux saturés ou insaturés de 5 ou 6 atomes ayant 1 à 4 atomes d'azote et/ou 1 ou 2 atomes d'oxygène ou de soufre, sous réserve que le nombre total d'hétéro-atomes soit au plus égal à 4; ou bien des noyaux bicycliques dans lesquels le noyau à 5 ou 6 chaînons est soudé à un noyau benzénique et le noyau bicyclique est relié par l'intermédiaire d'un atome de carbone du noyau benzénique, chaque noyau comprenant des substituants choisis entre les atomes et groupements alkyle, arylalkyle, diarylalkyle, alkylthio, alcoxy, halogène, nitro, oxo, cyano, hydroxy, amine, alkylamine, dialkylamine, trifluorométhyle, isocyanate, isothiocyanate et difluorométhoxy;

le terme "amine substitué" désigne un radical de formule $—NZ_1 Z_2$ dans laquelle $Z_1$ est l'hydrogène ou un radical alkyle ou aryl-$(CH_2)_m$— et $Z_2$ est un radical alkyle ou -aryl-$(CH_2)_m$—; ou bien $Z_1$ et $Z_2$ forment, avec l'atome d'azote auquel ilst sont fixés, un radical 1-pyrrolidinyle, 1-pipéridinyle, 1-azépinyle, 4-morpholinyle, 4-thiamorpholinyle, 1-pipérazinyle, 4-alkyl-1-pipérazinyle, 4-arylalkyl-1-pipérazinyle, 4-diarylalkyl-1-pipérazinyle, 1-pyrrolidinyle, 1-pipéridinyle, ou 1-azépinyle substitué par alkyle, alcoxy, alkylthio, halogène, trifluorométhyle ou hydroxy;

m est égal à 0 ou est un nombre entier de 1 à 6;

n est un nombre entier de 1 à 6; et

p est un nombre entier de 2 à 6.

2. Composé selon la revendication 1, dans lequel X est l'oxygène.

3. Composé selon la revendication 1, dans lequel X est le soufre.

4. Composé selon la revendication 1, dans lequel $R_2$ est le radical méthyle.

5. Composé selon la revendication 1, dans lequel $R_3$ est un radical alkyle.

6. Composé selon la revendication 1, dans lequel $R_4$ est le radical 3-nitrophényle.

7. Composé selon la revendication 1, dans lequel $R_1$ est un radical alkyle.

8. Composé selon la revendication 1, dans lequel $R_1$ est un radical alcényle.

9. Composé selon la revendication 1, dans lequel $R_1$ est un radical alcynyle.

10. Composé selon la revendication 1, dans lequel $R_1$ est un radical aryle.

11. Composé selon la revendication 1, dans lequel $R_1$ est un radical arylalkyle.

12. Composé selon la revendication 1, dans lequel $R_1$ est $(CH_2)_n—Y_2$ ou $—(CH_2)_p—Y_3$.

13. Composé selon la revendication 1, dans lequel $R_1$ est

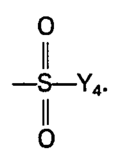

14. Composé selon la revendication 1, dans lequel X est le soufre, $R_2$ est le radical méthyle, $R_3$ est un radical alkyle et $R_4$ est le radical 3-nitrophényle.

15. Composé selon la revendication 1, servant à abaisser la pression sanguine chez un mammifère-hôte qui en a besoin.

16. Composé selon la revendication 1, servant de vasodilatateur bloquant l'entrée du calcium chez un mammifère-hôte qui en a besoin.